# EUROPEAN PATENT APPLICATION

(11) **EP 4 534 050 A2**
(43) Date of publication of application: **09.04.2025**
(21) Application number: 24212341.2
(22) Date of filing: 03.07.2020
(51) Int. Cl.: A61F 5/445

(54) **A SKIN CARE COMPOSITION**

(30) Priority: 03.07.2019 DK PA201970435
(62) Divisional of application: 20739261.4
(71) Applicant: Coloplast A/S, 3050 Humlebaek (DK)
(72) Inventor: Overgaard, Anne Kathrine Kattenhoej Sloth, 2830 Virum (DK); Gallego, Monica Ramos, 2450 Copenhagen SV (DK); Hoffmann, Christian, 2200 Copenhagen N (DK); Morse, Admira, 2200 Copenhagen N (DK)

(57) **Abstract**

A skin care composition comprising at least 1 weight % potato protein, a first binding agent, comprising polar or non-polar polymer or co-polymer(s) in an amount of 20-70 weight % and 20-80 weight % of a solid. The skin care composition is capable of interacting with enzymes in stomal output and thereby reducing the enzymatic activity.

## Description

### TECHNICAL FIELD

A skin care composition is provided, which is capable of reducing enzymatic activity in stomal output.

### BACKGROUND

Ostomy patients often use skin care compositions, to treat or prevent damage to the peristomal skin. Skin care compositions may be incorporated into stomal devices or be used as separate compositions e.g. in the form of lotions, creams or gels.

Stomal output contains enzymes, which are known to damage skin. These enzymes may be addressed by compositions that interacts with the enzyme and thereby reduces enzyme activity. In a process where enzymes are removed from a mixture e.g. by interacting with a compound, these enzymes will be unable to cause skin damage.

The peristomal region is characterised by mechanical stress due to the movements of the patient as well as sweat and body heat. Additionally, stomal output provides an environment with high enzyme activity. Skin care compositions should be formulated to handle such environmental factors, while maintaining good skin care properties. Additionally, skin care compositions should avoid excessive absorption of water which might increase enzyme concentration in the output.

### SUMMARY

A skin care composition is thus provided, comprising: at least 15 weight % potato protein, a first binding agent, comprising polar or non-polar polymer or co-polymer(s) in an amount of 20-50 weight % and 20-65 weight % of a solid, wherein the total content of potato protein, clay, and other solids is in the range of 50-75 weight %.

The use of a skin care composition as defined herein in a peristomal environment is also provided. A particular use of the skin care composition is for reducing enzymatic activity, in particular trypsin activity in stomal output.

A method for preventing skin damage and/or maceration is provided. A method for reducing enzymatic activity in stomal output is provided.

An ostomy device comprising the skin care composition as defined herein is also provided. A baseplate for an ostomy device, comprising the skin care composition as defined herein, is provided.

### DETAILED DISCLOSURE

In embodiments, a skin care composition is thus provided, comprising:
a. at least 1 weight % potato protein,
b. a first binding agent, comprising polar or non-polar polymer or co-polymer(s) in an amount of 20-70 weight %
c. 20-65 weight % of a solid comprising a clay.

When exposed to stomal output or water, the clay is washed out from the skin care composition and into the surroundings, where it can interact with enzymes from the stomal output and thereby reducing the enzyme activity.

When designing the skin care composition, the present inventors found it necessary to balance the properties of (a) texture of the composition; (b) expansion (swelling) of the composition upon contact with moisture; (c) release of clay from the composition; and (d) enzyme activity reducing effect of the composition. All these properties are affected by the amount of potato protein and clay in the composition.

The present inventors found that the total content of potato protein, clay, and other solids should be in the range of 50-75 % (w/w) in order to get an acceptable texture. Lower than 50 % (w/w) yielded too soft and flowable compositions while above 75 % (w/w) yielded too hard compositions.

The present inventors further found that a minimum of 20 % (w/w) of clay was beneficial to achieve the desired enzyme activity reduction. Adding more clay would increase the enzyme activity reduction but became problematic because of increased swelling of the composition when exposed to moisture. To counter this undesired swelling, the present inventors turned to potato protein. It was surprisingly found that adding potato protein made it possible to control the swelling of the composition without compromising the enzyme reducing activity. In fact, it was surprisingly found that the potato protein not only limited the swelling caused by the clay but also contributed to a slower and more steady release of clay from the composition, leading to better control of the enzyme reduction activity of the composition. In aspects, the invention relates to a skin care composition comprising at least 15 weight % potato protein, a first binding agent, comprising polar or non-polar polymer or co-polymer(s) in an amount of 25-50 weight % and 20-60 weight % of a solid comprising a clay, wherein the total content of potato protein, clay, and other solids is in the range of 50-75 weight %.

In embodiments, the skin care composition has a texture that allows for application to an ostomy appliance, where the composition may stay in place without flowing during storage but at the same time is able to be eroded and the potato protein and optionally the solids being washed out when exposed to stomal output. In embodiments, the composition has a dough-like texture, allowing the composition to be moulded into a desired configuration, such as a layer or coating, but is yet soft enough to follow the movements of the device. A challenge, when having a mixture of a powder/particles and one or more binders, is to avoid syneresis of the resulting composition. Syneresis is the process in which a gel contracts on standing and exudes liquid, as in the separation of whey in cheese-making.

In embodiments, the skin care composition has a low expansion when wetted. When the skin care composition is located close to the stoma, a large expansion of the composition during exposure to stomal output may give rise to unwanted pressure towards the stoma. The presence of potato protein in the composition may delay the wetting of the composition and thereby increase the time before the composition is washed away. Furthermore, the swelling of the clay may proceed slower when the potato protein is present.

The skin care composition is suitably non-adhesive, so that it does not interfere or adhere to other components of the ostomy device.

In embodiments, it is desired to have a composition that is coherent, has a texture that is not susceptible to syneresis or may flow during storage, show limited expansion during absorption of stomal output and yet is able to let the potato protein and the clay being washed out during wear time. It is a delicate balance to design a composition with these properties.

By "output" is meant the effluent from a stoma, being faeces and/or urine in a more or less viscous form, possibly together with mucins secreted from the epithelial layer of the alimentary canal. In the case of a colostomy, the output might be quite solid, whereas an ileostomy may produce more liquid output. The output may contain digestive fluids with enzymes and other components that may be aggressive to the skin and thus may cause maceration and contact dermatitis of the skin. The output may also comprise components which may attack and degrade the ostomy device itself, e.g. the adhesive.

By potato protein is herein meant protein obtained from potato tubers (Solanum tuberosum). Potato protein is extracted from potatoes and fractioned into fractions of different molecular weight. A fraction of low molecular weight contains potato protein capable of interacting with enzymes and reducing their activity. The potato protein is capable of interacting with enzymes, especially trypsin, an enzyme that is often present in stomal output where it may cause skin breakdown, leading to complications such as redness, itching and discomfort.

The skin care composition may comprise 15-60 weight %, such as 20-50 weight %, such as 25-40 weight % or even 25-35 weight % or 25-45 weight %, such as 30-45 weight % potato protein. In embodiments, at least 40 weight % of the total amount of particles (the combined amounts of potato protein, clay and other solids) in the composition is comprised of potato protein.

Potato protein shows little or no expansion when wetted and will thus not give rise to swelling/expansion of the skin care composition.

By "solids" is herein meant one or more components having a solid character, such as fillers, stabilizers etc. The skin care composition comprises at least one solid. The solids may help to adjust the texture of the composition, stability as well as it may influence the ability of the composition to be eroded when contacted with stomal output. In embodiments, the solids comprise clay. The clay is a particulate phyllosilicate material. The clay may comprise charged alumosilicate layers and interlayer cations. The clay may be present in an amount of 20-80 weight %, such as 20-65 weight %, such as 20-50 weight %, such as 20-40 weight %, or in an amount of 25-70 weight %, such as 25-70 weight %, preferably 25-60 weight %, more preferably 30-50 weight % of the composition. The clay is suitably a smectite clay, preferably selected from the group consisting of montmorillonite, beidellite, nontronite, saponite and hectorite. The clay may be a hectorite-type smectite clay, preferably laponite, suitably with CAS no. 53320-86-8, CAS no. 64060-48-6 or combinations thereof. In chemical terms, the clay is suitably a lithium magnesium sodium silicate or a sodium magnesium fluorosilicate.

Laponite RD belongs to the BYK family of lithium magnesium sodium silicates, which is the international nomenclature of cosmetic ingredients (INCI) name, which includes also laponite XLG and D. The empirical formula for laponite RD is Na⁺_{0.7}[(Si₈Mg_{5.5}Li_{0.3})O₂₀(OH)₄]^{-0.7}.

Laponite XL21 belongs to the INCI family of sodium magnesium fluorosilicate (CAS 64060-48-6). This material contains fluorine (F⁻) additionally to hydroxyl groups (OH⁻). The empirical formula for laponite XL21 is Na⁺_{0.7}[(Si₈Mg_{5.5}Li_{0.3})O₂₀(OH)_{2.5}F_{1.5}]^{-0.7}.

In embodiments, the solid comprises potato starch, talc, kaolin, florisil, Calcium Alginate, fumed silica, calcium carbonate, sodium bicarbonate, baking powder, citric acid, gelatine, blanose, microfibers, polymers such as polyacrylates, PVP, PVOH, PVA, polyolefins and co-polymers. In embodiments, the composition comprises 20-80 weight %, such as 25-60 weight %, or 20-30 weight %, or 40-50 weight % of potato starch.

In embodiments, the combined amount of potato protein, clay and other solids constitutes 50-75 weight %, such as 55-70 weight % of the composition. If the total amount of particles is too low, the texture of the composition may be too fluent. If the amount of particles is too high, the texture may be too hard and inflexible.

In embodiments, combination of potato protein and clay can yield a low expansion when wetted. This may in part be due to the low expanding nature of potato protein, but also to the effect that the swelling of the clay seems to be delayed in the presence of potato protein as the liquid may penetrate the composition slower. In a similar way, other solids used in the composition also have a low expansion when wetted. When the skin care composition is located close to the stoma, a large expansion of the composition during exposure to stomal output may give rise to unwanted pressure towards the stoma.

Some solids, such as clay, may also be capable of interacting with harmful constituents from the stomal output. Such materials may have an inherited ability of interacting with a broad spectre of compounds and may be a supplement to the effect of the potato protein which addresses more specifically the enzymes of the stomal output.

By combining potato protein and clay in the skin care composition, a composition capable of interacting a broad number of harmful substances from stomal output is achieved. The clay may be able to interact with a broad spectre of harmful substances whereas the potato protein is capable of interacting with enzymes. Enzymes, such as trypsin from the stomal output, can be rather aggressive when brought into contact with the peristomal skin and may cause damage to the skin. Furthermore, using clay alone for reducing the enzyme activity, a large expansion of the skin care composition, due to the presence of clay, may be achieved. By substituting at least a part of the clay with potato protein, a composition with low expansion, good texture and capable of reduce activity of harmful substances in the output is provided.

In embodiments, the skin care composition comprises solids having stabilizing properties. Stabilizers may prevent syneresis by stabilizing the texture of the skin care composition.

In embodiments, the solids comprise compounds facilitating easier and/or faster erosion of the skin care composition. Such compounds may enable a faster or more effective erosion of the potato protein and clay from the skin care compound. An example of such compound may be baking powder.

The skin care composition further comprises at least one binding agent(s). The binding agent(s) may be polar and/or non-polar polymer(s) or co-polymer(s) and may be present in an amount of 20-70 weight %, such as 20-50 weight %, such as 25-40 weight %.

In embodiments, the skin care compound comprises a first and a second binding agent which may work in tandem to ensure the correct texture of the skin care composition, and simultaneously the interaction of the potato protein and the clay upon contact with stomal output. The skin care composition may be in the form of a powder, a dough or a fluid depending on the ratios of solids and binders.

In embodiments, a polar binding agent comprises a compound being fluid at room temperature. In embodiments, the polar binding agent comprises glycerol, polyglycerols, polymers or co-polymers of ethylene glycol and/or polymers or co-polymers of propylene glycol.

Including an amount of a polar binding agent in the skin care composition may prevent syneresis of the skin care composition. In embodiments, the polar binding agent is present in the skin care composition in an amount of 10-70 weight %, such as 10-50 weight %, such as 20-40 weight %.

In embodiments, the skin care composition comprises a non-polar binding agent which is fluid at room temperature. The non-polar binding agent may be a polyolefin such as polybutene, hydrogenated polybutene, liquid isoprene, poly-alfa-olefin or wax. The non-polar binding agent may be present in an amount of 10-70 weight %, such as 20-50 weight%, such as 20-40 weight % of the composition.

In embodiments, the composition comprises a first non-polar binding agent having a molecular weight different from a second binding agent. Having a low molecular weight polymer together with a high molecular weight, syneresis of the skin care composition may be prevented.

In one particular aspect, the skin care composition comprises the following composition:
a. 10 - 50 weight % potato protein;
b. 25 - 50 weight % of a non-polar binding agent;
c. 35 - 50 weight % solid;

wherein said potato protein, non-polar binding agent are as defined above. In another particular aspect, the skin care composition comprises the following composition:
   a. 20 - 40 weight % potato protein;
   b. 25-45 weight % solid in the form of clay;
   c. 25- 40 weight % first non-polar binding agent;
   d. 1- 5 weight % second non-polar binding agent;
wherein said potato protein, clay, first binding agent and second binding agent are as defined above.

Embodiments provide a skin care composition comprising: 25-45 weight % potato protein; first binding agent comprising polar or non-polar polymer or co-polymer(s) in an amount of 20-50 weight %; and 20-40 weight % of a solid comprising a clay.

Embodiments provide a skin care composition comprising a combined amount of potato protein and solids in the composition of 50-75 weight % and comprising 25-45 weight % potato protein; first binding agent comprising polar or non-polar polymer or co-polymer(s) in an amount of 20-50 weight %; and 20-40 weight % of a solid comprising a clay.

The skin care composition may have one of the following compositions a - f, listed in Table 1:

**TABLE 1**

| **Composition** | **Potato protein (weight%)** | **Clay (weight%)** | **Other solids (weight %)** | **First binding agent (weight %)** | **Second binding agent (weight %)** |
|---|---|---|---|---|---|
| a. | 32 | | 38 | 28 | 2 |
| b. | 15 | | 52 | 33 | 10 |
| c. | 32 | | 37 | 29 | 2 |
| d. | 33 | 33 | | 33 | 1 |
| e. | 31 | 31 | 1 | 35 | 2 |
| f. | 30 | 40 | | 30 | |

Also provided is the use of a skin care composition as described herein in a peristomal environment. The skin care composition is used for reducing enzymatic activity, in particular trypsin activity. As mentioned, particles of potato protein and clay may be washed out from the skin care composition and interact with the enzymes.

A method for reducing enzymatic activity in output is provided. The method comprises the steps of providing a skin care composition as defined herein and applying the skin care composition to peristomal skin and/or to an ostomy wafer. Hereby, the skin care composition is allowed to reduce enzymatic activity in the stomal output that may come into contact with the composition.

A method for preventing skin damage and/or maceration is provided. The method comprises the steps of providing a skin care composition as defined herein and applying the skin care composition to peristomal skin and/or to an ostomy wafer. Hereby, the skin care composition is allowed to reduce enzymatic activity in any stomal output that might come into contact with the peristomal skin, thereby reducing the aggressiveness of the output and preventing skin damage.

An ostomy device is also provided comprising the skin care composition described herein, as well as a baseplate for an ostomy device, comprising the skin care composition described herein.

### EXAMPLE

A skin care composition as disclosed in Table 2 below is mixed. The composition has a dough-like texture, low expansion/swelling and shows good cohesion with no signs of syneresis. When exposed to stomal output, a reduction in enzyme activity in the output is achieved.

**TABLE 2**

| Component | % weight |
|---|---|
| Indopol H300 | 28,3 |
| Oppanol B10 SFN | 1,7 |
| Laponite | 32,5 |
| Potato protein | 32,5 |

## Claims

1. A method for reducing enzymatic activity in output, comprising the steps of:
A) of providing a skin care composition comprising
a. at least 15 weight % potato protein,
b. 25-50 weight % of a first binding agent comprising polar or non-polar polymer or co-polymer(s), and
c. 20-60 weight % of a solid comprising a clay,
wherein the total content of potato protein, clay, and other solids is in the range of 50-75 weight % and
B) applying the skin care composition to peristomal skin and/or to an ostomy wafer.

2. The method of claim 1, wherein said provided skin care composition, wherein the composition comprises 15-60 weight %, such as 20-50 weight %, such as 25-40 weight % or even 25-35 weight % of potato protein.

3. The method of any one of the preceding claims, wherein said provided skin care composition, wherein the composition comprises a second binding agent having a molecular weight being different from the first binding agent.

4. The method of any one of the preceding claims, wherein said provided skin care composition according to any one of the preceding claims, wherein said clay is a smectite clay, preferably selected from the group consisting of montmorillonite, beidellite, nontronite, saponite and hectorite.

5. The method of any one of the preceding claims, wherein said provided skin care composition, wherein the solid comprises potato starch or talc.

6. The method of any one of the preceding claims, wherein said provided skin care composition is allowed to reduce enzymatic activity in the stomal output that may come into contact with the composition.

7. The method of any one of the preceding claims, wherein said provided skin care composition is allowed to reduce trypsin activity in the stomal output that may come into contact with the composition.

8. A method for preventing skin damage and/or maceration in output, comprising the steps of:
A) of providing a skin care composition comprising
a. at least 15 weight % potato protein,
b. 25-50 weight % of a first binding agent comprising polar or non-polar polymer or co-polymer(s), and
c. 20-60 weight % of a solid comprising a clay,
wherein the total content of potato protein, clay, and other solids is in the range of 50-75 weight % and
B) applying the skin care composition to peristomal skin and/or to an ostomy wafer.

9. The method of claim 8, wherein said provided skin care composition, wherein the composition comprises 15-60 weight %, such as 20-50 weight %, such as 25-40 weight % or even 25-35 weight % of potato protein.

10. The method of any one of claims 8-9, wherein said provided skin care composition, wherein the composition comprises a second binding agent having a molecular weight being different from the first binding agent.

11. The method of any one of claims 8-10, wherein said provided skin care composition according to any one of the preceding claims, wherein said clay is a smectite clay, preferably selected from the group consisting of montmorillonite, beidellite, nontronite, saponite and hectorite.

12. The method of any one of claims 8-11, wherein said provided skin care composition, wherein the solid comprises potato starch or talc.

13. The method of any one of claims 8-12, wherein said provided skin care composition is allowed to reduce enzymatic activity in any stomal output that might come into contact with the peristomal skin, thereby reducing the aggressiveness of the output and preventing skin damage.

14. The method of any one of claims 8-13, wherein said provided skin care composition is allowed to reduce trypsin activity in any stomal output that might come into contact with the peristomal skin, thereby reducing the aggressiveness of the output and preventing skin damage.
